# EUROPEAN PATENT APPLICATION

(11) **EP 3 964 820 A1**
(43) Date of publication of application: **09.03.2022**
(21) Application number: 20194866.8
(22) Date of filing: 07.09.2020
(51) Int. Cl.: G01N 21/35

(54) **NUTRIENT CONTENT DETERMINATION IN A LIQUID FOOD PRODUCT**

(71) Applicant: Koninklijke Philips N.V., 5656 AG Eindhoven (NL)
(72) Inventor: RONDA, Cornelis Reinder, 5656 AE Eindhoven (NL); VAN DEN ELZEN, Stef, 5656 AE Eindhoven (NL); KOOIJMAN, Gerben, 5656 AE Eindhoven (NL)
(74) Representative: Philips Intellectual Property & Standards

(57) **Abstract**

A near-IR (infrared) spectrophotometric system (10) is disclosed for detecting the content of at least one nutrient type in a liquid food product (1). Each nutrient type exhibits a plurality of near-IR absorptions (31) at wavelengths characteristic of said nutrient type upon exposure to near-IR radiation (21) comprising said wavelengths. The near-IR spectrophotometric system comprises a near-IR light source arrangement (20) comprising at least one near-IR light source (22-25) for generating the near-IR radiation; and a near-IR detector arrangement (30) comprising at least one near-IR detector (32-35) for detecting the near-IR radiation reflected by the liquid food product. The near-IR spectrophotometric system is configured such that the near-IR detector arrangement exclusively detects at least one set of discrete spectral portions of said near-IR radiation, the discrete spectral portions within each set containing respective absorption wavelengths characteristic of a particular one of said at least one nutrient type. A vessel, electronic device and food processing apparatus comprising such a near-IR spectrophotometric system (10) are also disclosed.

## Description

### FIELD OF THE INVENTION

The present invention relates to a near-IR (infrared) spectrophotometric system for detecting the content of at least one nutrient type in a liquid food product.

The present invention further relates to a vessel for containing a liquid food product comprising such a near-IR spectrophotometric system.

The present invention further relates to food processor comprising such a vessel.

The present invention further relates to a portable electronic device comprising such a near-IR spectrophotometric system.

### BACKGROUND OF THE INVENTION

There is an increasing demand in modem society to obtain information regarding the nutritional composition of food products, for example to maintain a balanced diet, limit the intake of particular nutrient types, and so on. To this end, smart kitchen appliances such as blenders have been produced that can estimate the nutritional value information for a particular food product from predefined recipe information made available for that food product. However, such nutritional value information can be inaccurate, in particular when the consumer deviates from the predefined recipe, e.g. by changing the ratio of ingredients or adding ingredients that are not part of the predefined recipe. This is particularly relevant for liquid food products, e.g. blended products such as smoothies, juices and so on.

A promising technique for food product evaluation is near-IR reflectance spectroscopy, which is based on the principle that most compounds have vibrational overtones that absorb a broad spectrum of near-IR radiation. Recently, a compact spectrophotometer under the name SCiO^{®} has been marketed by Consumer Physics, Inc., St. Cloud, Minnesota, USA, which is a handheld device using near-IR reflectance spectroscopy to determine the nutritional content of a food product in term of nutrient types such as carbohydrates, fats and proteins. However, the form factor and cost of this spectrophotometer precludes its integration into portable electronic devices such as smart phones or food processing apparatuses such as blenders and juicers. Hence, there is a need for a more compact near-IR spectrophotometer that can accurately determine the nutrient type content in food products, in particular liquid food products.

### SUMMARY OF THE INVENTION

The present invention seeks to provide a near-IR (infrared) spectrophotometric system for detecting the content of at least one nutrient type in a liquid food product that is compact enough such that it can be integrated in a range of devices in a cost-effective manner.

The present invention further seeks to provide a vessel for containing a liquid food product comprising such a near-IR spectrophotometric system.

The present invention further seeks to provide a food processing apparatus comprising such a vessel.

The present invention further seeks to provide a portable electronic device comprising such a near-IR spectrophotometric system.

According to an aspect, there is provided a near-IR (infrared) spectrophotometric system for detecting the content of at least one nutrient type in a liquid food product, each nutrient type generating a plurality of near-IR absorptions at wavelengths characteristic of said nutrient type upon exposure to near-IR radiation comprising said wavelengths, the near-IR spectrophotometric system comprising a near-IR light source arrangement comprising at least one near-IR light source for generating the near-IR radiation; and a near-IR detector arrangement comprising at least one near-IR detector for detecting the near-IR radiation reflected by said liquid food product; said near-IR spectrophotometric system being configured such that the near-IR detector arrangement exclusively detects at least one set of discrete spectral portions of said near-IR radiation, the discrete spectral portions within each set containing respective absorption wavelengths characteristic of a particular one of said at least one nutrient type.

The present invention is based on the insight that although near-IR overtone absorptions are typically spectrally broad, narrowband absorption regions, i.e. specific absorption wavelengths, within such overtone absorptions not only can be specific to particular nutrient types, but can also be used to accurately determine the content of such nutrient types within a liquid food product. Moreover, the size of the near-IR spectrophotometric system can be effectively reduced by limiting the generation and/or detection of wavelengths within the near-IR spectrum, e.g. a spectrum from 800-2500 nm and more preferably from 800-1100 nm, to only those wavelengths at which the nutrient types of interest exhibit optical absorptions that are characteristic of that nutrient type. This significantly reduces the complexity of the near-IR spectrophotometric system and allows for the low-cost manufacture of a system that can be fitted into a volume as little as 1cm³, thereby facilitating integration of the system in a wide range of objects, as will be explained in further detail below.

For example, the near-IR light source arrangement may comprise a light source arranged to generate a continuous near-IR radiation spectrum including said wavelengths and the near-IR detector arrangement may comprise a plurality of detectors, each of said detectors being arranged to detect one of said discrete spectral portions. This significantly simplifies the detector architecture, thus enabling the desired compactness of the near-IR spectrophotometric system.

In an embodiment, the detectors are arranged on a common substrate such as a silicon substrate, for example where the detectors are manufactured in a cost-effective manner using routine semiconductor manufacturing technology.

Alternatively or additionally, the near-IR light source arrangement comprises a plurality of light sources each arranged to generate one of said discrete spectral portions, and the near-IR detector arrangement comprises a detector arranged to detect each of said discrete spectral portions in order to achieve the exclusive generation of narrowband spectral portions containing the characteristic reflectance wavelengths of the nutrient types of interest.

In an embodiment, said light sources are narrowband light emitting diodes or lasers, which allows for a particular compact and cost-effective implementation of the near-IR spectrophotometric system.

Each discrete spectral portion may have a bandwidth of less than 2 nm, preferably less than 1 nm to ensure a high resolution near-IR spectrophotometric system in which characteristic absorption wavelengths can be detected in isolation.

Preferably, the total number of discrete spectral portions in each set does not exceed four. It has been found that for the nutrient types of interest, the content of such nutrient types within a liquid food product can be accurately determined using no more than four characteristic absorption wavelengths, such that limiting each set to no more than four characteristic absorptions translates into a particularly compact near-IR spectrophotometric system.

In an example embodiment, the at least one nutrient type comprises at least one of carbohydrates, sugar, fiber fat and protein and the discrete spectral portions in the set for carbohydrates respectively contain the absorption wavelengths at 860nm, 878nm, 887nm, and/or 919nm characteristic for carbohydrates; the discrete spectral portions in the set for sugar respectively contain absorption wavelengths at 935nm, 937nm, 940nm, and/or 948nm characteristic for sugar; the discrete spectral portions in the set for fiber respectively contain absorption wavelengths at 872nm, 891nm, 903nm, and/or 912nm characteristic for fiber; the discrete spectral portions in the set for fat respectively contain absorption wavelengths at 829nm, 931nm, and/or 939nm; and/or characteristic for fat; and the discrete spectral portions in the set for protein respectively contain absorption wavelengths at 1028nm, and/or 1049nm characteristic for protein. Preferably, the near-IR spectrophotometric system is adapted to detect each of these nutrient types. For the avoidance of doubt, where reference is made to sugars, these are simple carbohydrates that typically lack an acyclic ether moiety within their molecular structure, e.g. monosaccharides and disaccharides, whereas where reference is made to carbohydrates, these are complex carbohydrates, e.g. oligosaccharides and polysaccharides comprising one or more of such acyclic ether moieties.

The near-IR detector arrangement may be further adapted to detect a set of discrete further spectral portions, each of said further spectral portions containing a spectral absorption wavelength characteristic for water. This has the advantage that the absolute content of the nutrient types of interest may be determined in the liquid food product, for example where the total weight or total volume (and density) of the liquid food product is known.

The near-IR spectrophotometric system may comprise a data communication module communicatively coupled to the near-IR detector arrangement such that the spectral data collected by the near-IR detector arrangement can be communicated to a remote device for analysis. Alternatively, the near-IR spectrophotometric system may further comprise a processor communicatively coupled to the near-IR detector arrangement and adapted to determine the content of the at least one nutrient type from the respective intensities of the absorptions measured by the near-IR detector arrangement at the wavelengths characteristic of said nutrient type such that the near-IR spectrophotometric system can operate as a self-contained system.

In an embodiment in which a particular measured absorption comprises an overlap of a first absorption at a first wavelength characteristic of a first nutrient type and a second absorption at a second wavelength characteristic of a second nutrient type, the processor is adapted to determine the content of the first nutrient type from measured absorptions at wavelengths characteristic of said first nutrient type other than said particular measured absorption; scale the particular measured absorption by subtracting the contribution of the first wavelength characteristic of a first nutrient type from said particular measured absorption using the determined content of the first nutrient type; and determine the content of the second nutrient type from measured absorptions at wavelengths characteristic of said second nutrient type including said scaled particular measured absorption. This for instance may be useful where the content of the second nutrient type cannot be accurately determined without such differential analysis of the particular measured absorption, e.g. because the second nutrient type only exhibits a few characteristic absorptions.

The processor may further be adapted to receive at least one of weight information and volume information for said liquid food product and further determine the content of the at least one nutrient type in said liquid food product based on said received weight information and/or volume information, e.g. to determine the absolute content of the nutrient type(s) of interest rather than their relative content in the liquid food product. The processor may further be adapted to receive nutrient type selection information and determine the content of the at least one nutrient type in said liquid food product identified in said nutrient type selection information. This further instance is useful where a user is interested in the content of a particular nutrient type in the liquid food product only, e.g. sugar for a diabetic patient, such that only this information is produced by the processor.

According to another aspect, there is provided a vessel for containing a liquid food product, the vessel comprising the near-IR spectrophotometric system of any of the herein described embodiments for detecting the nutrient content of the liquid food product within the vessel. Such a smart vessel, e.g. a smart cup, beaker, jug or the like, for example may be used in conjunction with a portable electronic device such as a smart phone or the like for processing the spectral information collected with the near-IR spectrophotometric system within the vessel such as to provide the nutrient content of the liquid food product within the vessel.

According to another aspect, there is provided a food processing apparatus for processing a liquid food product, the food processing apparatus comprising the vessel of any of the herein described embodiments, wherein said vessel is a food processing chamber. Such a smart food processing apparatus, e.g. a blender or a juicer, can provide an accurate composition of a liquid food product contained within the food processing chamber without the need for accurate recipe information.

According to another aspect, there is provided a portable electronic device comprising the near-IR spectrophotometric system of any of the herein described embodiments for detecting the nutrient content of the liquid food product within a vessel. Such a portable device may be used to evaluate the nutrient content of a liquid food product such as a juice or smoothie in a simple and straightforward manner.

### BRIEF DESCRIPTION OF THE DRAWINGS

Embodiments of the invention are described in more detail and by way of non-limiting examples with reference to the accompanying drawings, wherein:
FIG. 1 schematically depicts a near-IR spectrophotometric system according to an embodiment;
FIG. 2 schematically depicts a near-IR spectrophotometric system according to another embodiment;
FIG. 3 schematically depicts a near-IR spectrophotometric system according to an embodiment in more detail;
FIG. 4 schematically depicts a near-IR spectrophotometric system according to another embodiment in more detail;
FIG. 5 schematically depicts an aspect of a near-IR spectrophotometric system according to an embodiment in more detail;
FIG. 6 depicts a graph showing the accuracy of the content determination of nutrient types as a function of the number of characteristic near-IR absorption wavelengths taken into consideration;
FIG. 7 depicts a graph showing characteristic absorption wavelengths for a number of nutrient types;
FIG. 8 schematically depicts a food processing apparatus including a near-IR spectrophotometric system according to an embodiment; and
FIG. 9 schematically depicts a food processing apparatus including a near-IR spectrophotometric system according to another embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

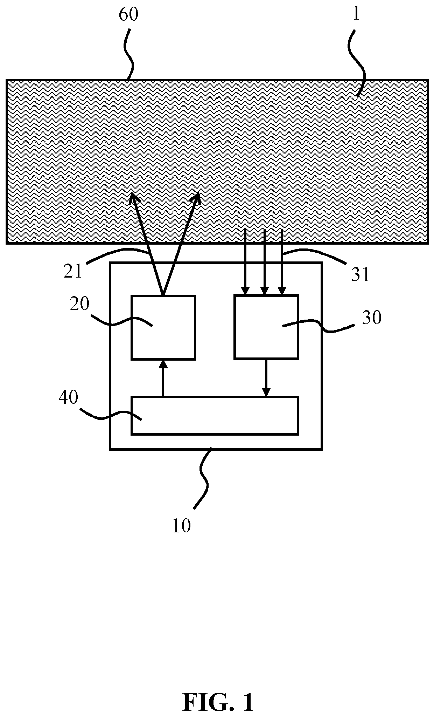

FIG. 1 schematically depicts a NIR (near-IR) spectrophotometric system 10 for determining the content of one or more nutrient types such as sugar, carbohydrates, fat, fiber and/or protein in a liquid food product 1 contained in a vessel 60. In an embodiment, the NIR spectrophotometric system 10 is adapted to detect sets of absorption wavelengths characteristic for each of these nutrient types. The NIR spectrophotometric system 10 comprises a light source arrangement 20 arranged to project light 21 having a known spectral composition within the NIR band of the electromagnetic spectrum, e.g. within the 800-2500 nm band, such as within the 800-1100 nm band, into the liquid food product 1. The NIR spectrophotometric system 10 further comprises a detector arrangement 30 arranged to detect NIR reflections 31 from the liquid food product 1. The liquid food product 1 typically is a cloudy liquid food product, i.e., has a transmittance of zero, such as in the case of smoothies and juices, such that the detected intensity of the NIR radiation at a particular wavelength by the detector arrangement 30 is indicative of the difference between the intensity of the NIR radiation generated by the light source arrangement 20 and the absorbance by the liquid food product 1 of that particular wavelength. The NIR spectrophotometric system 10 may further comprise a processor 40 adapted to determine the relative or absolute content of one or more nutrient types within the liquid food product from the intensities of reflected wavelengths that are characteristic for that particular nutrient type, i.e., the amount of light absorbed by such nutrients at such wavelengths, as will be explained in further detail below. The NIR spectrophotometric system 10 may be integrated in the vessel 60, may be a standalone system or may be integrated in another device, e.g. a portable electronic device such as a smart phone or tablet computer through which the nutrient content evaluation may be performed by aiming the NIR spectrophotometric system 10 at the liquid food product contained within the vessel 60. The vessel 60 may take any suitable shape, e.g. a cup, jug, beaker or the like.

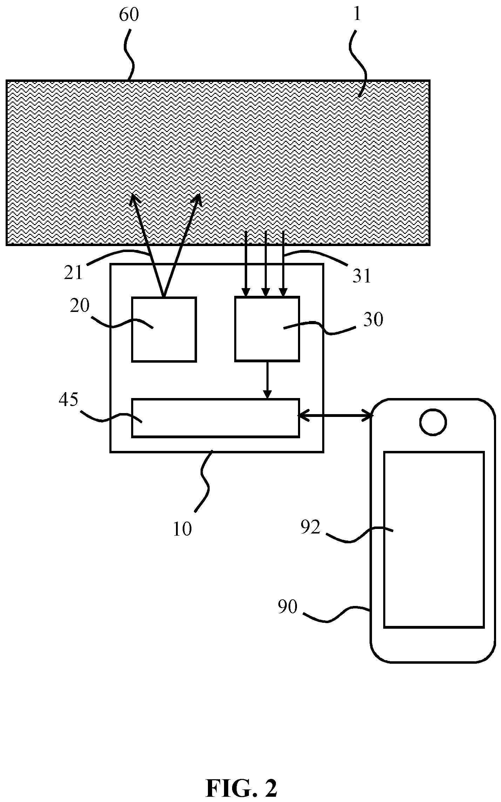

In addition to the processor 40 or instead of the processor 40, the NIR spectrophotometric system 10 may comprise a data communication module 45 as shown in FIG. 2, e.g. a wireless communication module such as a Bluetooth module, Wi-Fi module or the like, through which the NIR spectrophotometric system 10 may communicate data to a portable electronic device 90 such as a smart phone or tablet computer. For example, the NIR spectrophotometric system 10 may be communicatively coupled by the data communication module 40 to the portable electronic device 90 through a wireless communication link such as a Bluetooth link. The portable electronic device 90 may comprise the processor 40 adapted to determine the relative or absolute content of one or more nutrient types within the liquid food product from the measured intensities of reflected wavelengths that correspond to characteristic absorptions for that particular nutrient type and a display 92 onto which these processing results are displayed. Alternatively, the portable electronic device 90 may relay the spectral data received from the detector arrangement 30 to a remote processor such as a cloud-based service and receive the processing results from the remote processor for displaying on the display 92. In yet another embodiment, the NIR spectrophotometric system 10 may be integrated in the portable electronic device 90 as previously explained, in which the processor 40 and/or the data communication module 45 may be omitted from the NIR spectrophotometric system 10 as will be readily understood by the skilled person.

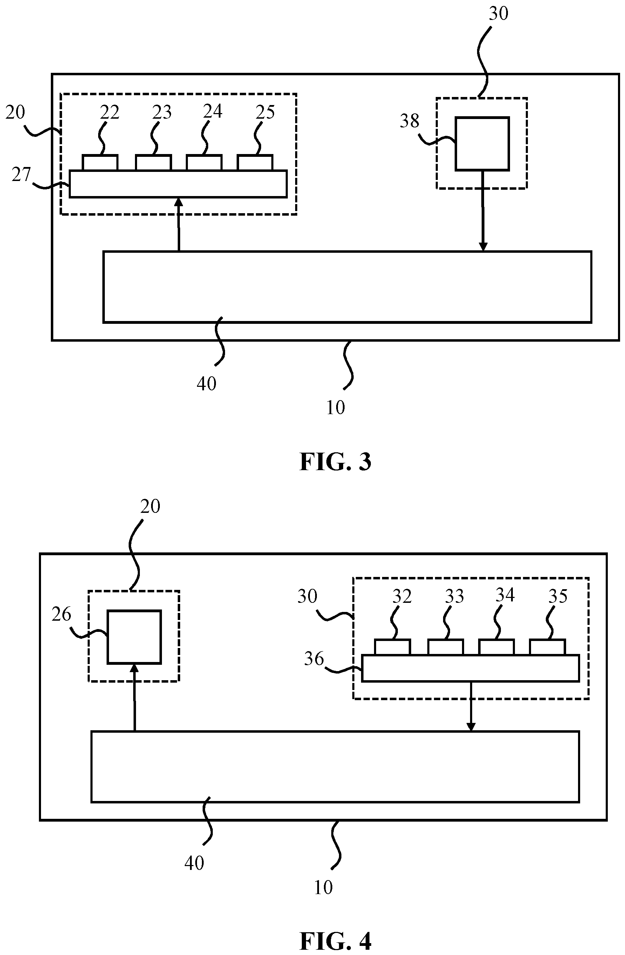

In accordance with the present invention, the NIR spectrophotometric system 10 is configured such that the detector arrangement 20 only detects reflectance at wavelengths that are characteristic absorption wavelengths for the one or more nutrient types of interest in order to reduce the complexity and size of the NIR spectrophotometric system 10. In a first embodiment, which is schematically depicted in FIG. 3, the light source arrangement 20 comprises a plurality of narrowband light sources 22-25, e.g. narrowband LEDs or lasers having an emission spectrum with a FWHM spectral width of less than 1 nm, with each of the light sources 22-25 being arranged to generate a different NIR spectral portion containing the characteristic absorption wavelength of one of the nutrient types of interest. Four light sources 22-25 are shown by way of non-limiting example only, and the skilled person will understand that the light source arrangement 20 may contain any suitable number of discrete narrowband light sources. The plurality of narrowband light sources 22-25 may be mounted on a common substrate 27, e.g. a silicon substrate, a carrier such as a PCB and so on. The detector arrangement 30 may comprise a broadband NIR detector 38 arranged to detect the reflections at said different wavelengths from the liquid food product 1 contained within the vessel 60.

In an alternative embodiment, which is schematically depicted in FIG. 4, the light source arrangement 20 comprises a broadband light source 26 for generating the NIR spectrum containing all the characteristic absorption wavelengths of the nutrient types of interest. Such a light source 26 is readily available and will therefore not be explained in further detail for the sake of brevity only. In case of a light source 26 generating a broadband spectrum with spectral components outside the NIR spectrum, e.g. UV-VIS components, the NIR spectrophotometric system 10 may further comprise a filter (not shown) for filtering out the components outside the NIR spectrum from the light output 21 to prevent undesired second order contributions to the reflection spectra.

In this embodiment, the detector arrangement comprises a plurality of narrowband detectors 32-35, with each detector being arranged to detect a different NIR spectral portion containing the characteristic absorption wavelength of one of the nutrient types of interest. Four detectors 32-35 are shown by way of non-limiting example only, and the skilled person will understand that the detector arrangement 30 may contain any suitable number of discrete narrowband detectors. For example, a detector array having a plurality of discrete detectors 32-35 may be manufactured on a common substrate or wafer 36 in a cost-effective manner, e.g. using semiconductor manufacturing techniques. As such techniques are well-known per se, this will not be explained in further detail for the sake of brevity only. The detectors 32-25 may be given narrowband detection characteristics by positioning appropriate interference or bandpass filters in front of the detectors 32-25, e.g. as discrete filter elements or as a film or coating over the light-sensitive surfaces of the detectors 32-35.

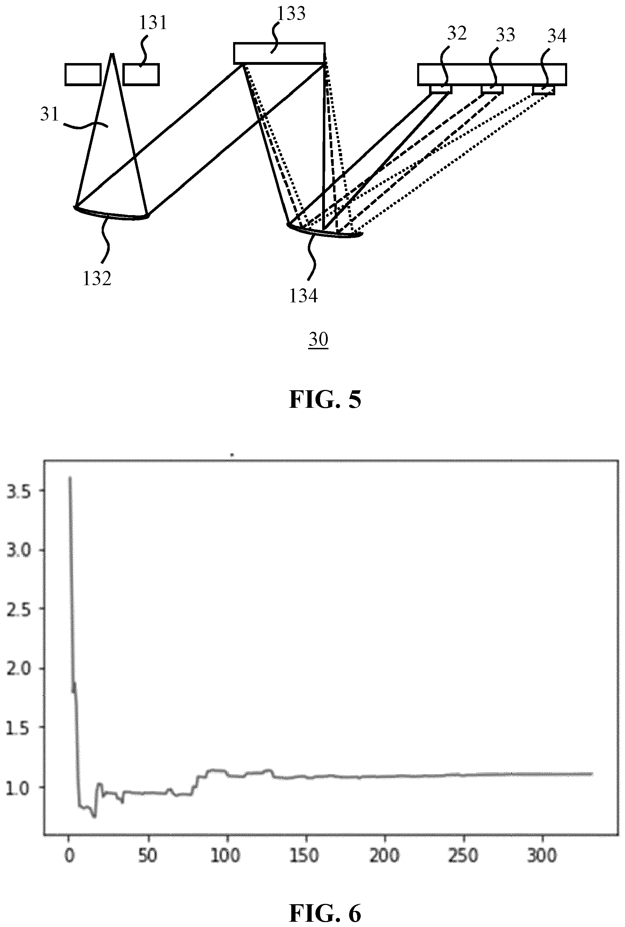

In order to direct light having the appropriate wavelength to each of the detectors 32-35, the detector arrangement 30 of the NIR spectrophotometric system 10 may be configured similar to a Czerny-Turner spectrometer as schematically depicted in FIG. 5, in which the NIR light reflected by the liquid food product 1 in the vessel 60 enters a housing of the detector arrangement 30 through a slit 131 and is redirected onto a diffraction grating 133 by a first mirror 132. The diffraction grating 133 spatially separates the various spectral components of the reflected NIR light, which spatially separated spectral components are redirected onto the detectors 32-34 by a second mirror 134. As such optical arrangements are well-known per se, this will not be explained in further detail for the sake of brevity only. It is noted that although FIG. 5 depicts three detectors 32-34 only, the skilled person will understand that this is by way of non-limiting example only and that any suitable number of spatially separated detectors may be present in the detector arrangement 30 as previously explained. Other suitable arrangements for separating out the spectral components of the reflected light incident on the detector arrangement 30 will be immediately apparent to the skilled person.

Although not explicitly shown, it is of course equally feasible to combine a light source arrangement 20 having a plurality of narrowband light sources 22-25 with a detector arrangement 30 having a plurality of narrowband detectors 32-35 in a NIR spectrophotometric system 10 according to embodiments of the present invention. By exclusively generating and/or detecting spectral components of NIR radiation having wavelengths at which the one or more nutrient types of interest exhibit absorption characteristic for that nutrient type, e.g. sugars, carbohydrates, fibers, fats and/or proteins, the size of the NIR spectrophotometric system 10 can be significantly reduced compared to many existing systems. For instance, by limiting the set of spectral portions containing wavelengths at which the aforementioned nutrient types exhibit absorption characteristics for that nutrient type to four of such spectral portions, a NIR spectrophotometric system 10 having a total volume of about 1 cm³ can be produced at low cost, e.g. 1-2 USD, which facilitates integration of such a NIR spectrophotometric system 10 in common objects such as the vessel 60, the portable electronic device 90 or a food processing apparatus 50 as will be described in further detail below.

In order to achieve accurate determination of the nutrient content of a liquid food product 1, the algorithm developed to process the spectral information collected by the detector arrangement 30 of the NIR spectrophotometric system 10 was trained using test samples of liquid food products 1 having well-defined nutrient contents to correlate the respective intensities of the reflectance detected at wavelengths exhibiting absorptions that are characteristic to the nutrient content in the test samples. In this calibration exercise, the number of characteristic absorption wavelengths that had to be taken into consideration in order to achieve an accurate determination of the nutrient content in the test sample was evaluated. The results of such an evaluation are shown in FIG. 6, which shows the cross validation score (Y-axis) as a function of the number of characteristic absorption wavelengths (X-axis) taken into consideration for carbohydrates. From these evaluations it was found that excellent accuracy of the nutrient determination could be achieved using no more than four data points, i.e. four characteristic absorption wavelengths, and that the addition of further characteristic wavelengths to the set of discrete spectral portions including such characteristic wavelengths to be detected by the detector arrangement 30 for such a nutrient did not significantly increase the accuracy of its content determination. This insight is a key facilitator in miniaturizing the form factor of the NIR spectrophotometric system 10, as for a system arranged to detect M nutrient types, in which M is a positive integer, at most 4*M wavelengths need to be generated and/or detected, thus limiting the required (silicon) real estate required.

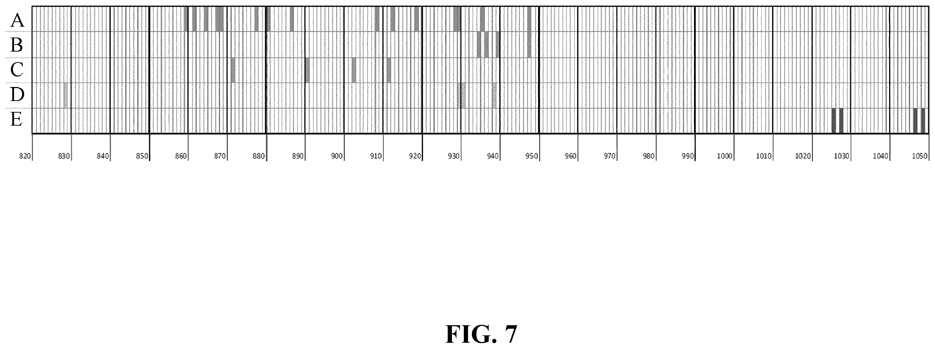

FIG. 7 depicts a set of preferred absorption wavelengths of the most common nutrient types of interest.

**Table 1.**

| Nutrient | Characteristic wavelength (nm) | | | |
|---|---|---|---|---|
| Carbohydrates | 860 | 878 | 887 | 919 |
| Sugars | 935 | 937 | 940 | 948 |
| Fiber | 872 | 891 | 903 | 912 |
| Fat | 829 | 931 | 939 | |
| Protein | 1028 | 1049 | | |

Table 1 lists selected preferred selection of absorption wavelengths characteristic for each nutrient type that may be included in the determination of the nutrient content in a liquid food product. It was found that using the characteristic wavelengths in Table 1, an accurate determination of the content of a liquid food product using a minimal set of data points could be achieved, thus allowing for the design of a particularly compact NIR spectrophotometric system 10. Table 1 further shows that for some nutrient types, e.g. fat and protein, fewer than 4 data points may need considering to accurately determine the nutrient content. Of course, additional and/or other characteristic wavelengths as shown in FIG. 6 may be used. Table 2 lists all the (characteristic) absorption wavelengths as shown in FIG. 6.

**Table 2**

| Nutrient | Wavelengths (nm) |
|---|---|
| Carbohydrates (A) | 860, 862, 865, 868, 869, 878, 881, 887, 909, 913, 919, 929, 930, 936, 948 |
| Sugars (B) | 935, 937, 940, 948 |
| Fiber (C) | 872, 891, 903, 912 |
| Fat (D) | 829, 930, 931, 939 |
| Protein (E) | 1026, 1028, 1047, 1049 |

In an embodiment, the algorithm may further be configured to also consider reflectance detected in spectral regions exhibiting non-characteristic absorptions, i.e. reflectance having absorption contributions from two nutrient types, such as at 930 nm for example, where both carbohydrates and fat exhibit absorptions. The algorithm may deploy differential analysis in such a scenario, where the contribution of one of the nutrient types to the composite absorption is determined from a sufficient number of characteristic data points at other wavelengths by determining the nutrient content from these other data points, after which this contribution is subtracted from the composite absorption to yield the contribution to the composite absorption by the other nutrient type. This for instance can be advantageous where the other nutrient type has too small a number of characteristic absorption wavelengths to accurately determine its content in the liquid food product, such that additional data points can be generated by including contributions from such composite absorption data points in order to improve the accuracy of its content determination.

In some embodiments, the algorithm is configured to determine the relative nutrient content of a liquid food product based on the spectral information collected by the detector arrangement 30. However, the processor 40 (or other applicable processor) running the algorithm may be configured to receive additional information such as the weight, volume, and so on, of the liquid food product, e.g. from dedicated sensors or through user input, to further improve the accuracy of the nutrient content determination and/or calculate the absolute nutrient content of the liquid food product, e.g. to accurately calculate its energy content. For example, where the NIR spectrophotometric system 10 is integrated in a food processing apparatus such as a blender or a juicer, the system may be configured to detect the amount of each ingredient as it is added to the food processing apparatus, e.g. by weighing or user specification, and use a default water content for each ingredient type in order to determine the water content in the liquid food product. Where additional water is added to the liquid food product in its preparation, the weight or volume of the additional water may be determined as previously explained to facilitate the accurate determination of the nutrient content determination and/or calculate the absolute nutrient content of the liquid food product. Alternatively, the detector arrangement 30 may be further adapted to detect a set of discrete further spectral portions, each of said further spectral portions containing an absorption wavelength characteristic for water such that the water content of the liquid food product can be accurately determined from this spectral information.

In a further refinement, the algorithm may be configurable such that only the content of selected nutrient types is determined. For example, the processor 40 may be responsive to a user interface through which a user may select for which nutrients the content in the liquid food product should be determined, such that the processor 40 configures the algorithm to only determine the contents of the nutrient type(s) specified by the user. This may be useful in a scenario in which a user is only interested in a particular nutrient type, e.g. sugar for controlling diabetes mellitus.

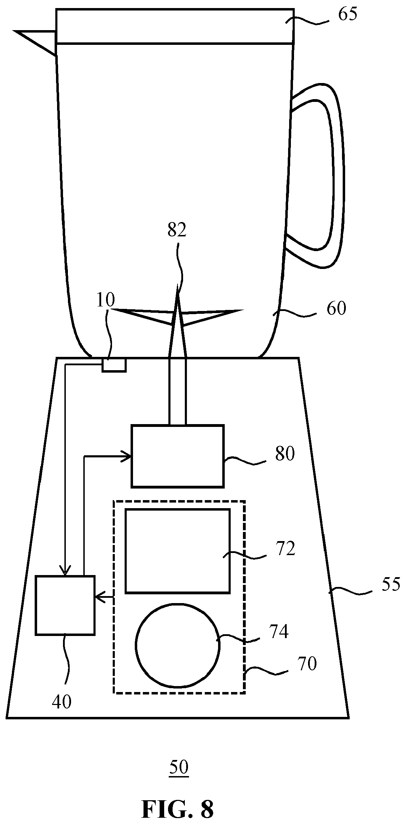

As previously mentioned, the NIR spectrophotometric system 10 may be integrated in a food processing apparatus for preparing liquid food products, such as a blender or a juicer. Other types of food processing apparatuses are of course equally feasible. An example embodiment of such a food processing apparatus 50 is shown in FIG. 8, in which the vessel containing at least the optical cell of the NIR spectrophotometric system 10 containing the light source arrangement 20 and the detector arrangement 30 is the food processing chamber 60 of the food processing apparatus 50, e.g. a jug or the like, having a lid 65 and a blade arrangement 82 within the food processing chamber 60 driven by a motor 80 under control of the processor 40 or a separate controller (not shown) within a housing 55 of the food processing apparatus 50. The processor 40 or separate controller may be responsive to a user interface 70, which may take any suitable shape or form, e.g. a knob or dial 74 for regulating the operation of the motor 80 and a display 72 for displaying operation menu options, information such as the nutrient content of a liquid food product detected with the NIR spectrophotometric system 10 etcetera. It should be understood that this is a non-limiting example of the user interface 70, which may take any suitable shape or form.

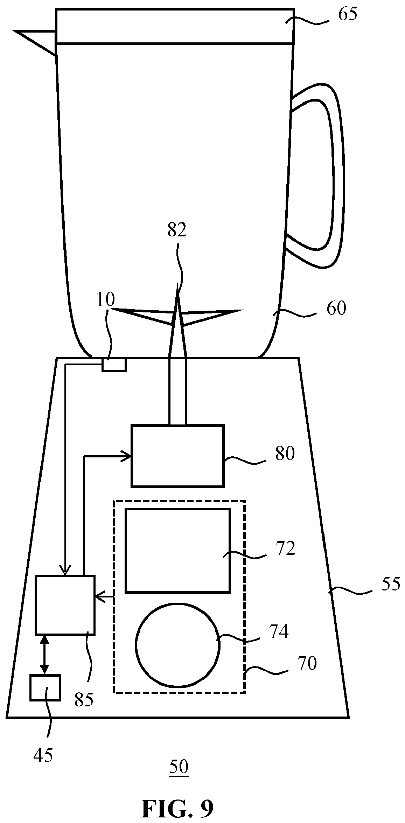

As should be understood from the foregoing, the NIR spectrophotometric system 10 may be implemented without the processor 40, in which case the processor 40 may be replaced by a controller 85 as schematically depicted in FIG. 9 that further controls a data communication module 45 for communicating the spectral information collected by the detector arrangement 30 of the NIR spectrophotometric system 10 to a remote device such as the portable electronic device 90 for remote processing, e.g. by the portable electronic device 90 or a cloud service as previously explained, after which the processing results, i.e. the nutrient content evaluation results may be made available on the portable electronic device 90 or on the display 72 of the user interface 70. Alternatively, the data communication module 45 may directly connect to the cloud service, e.g. over the Internet or the like, in order to provide the cloud service with the collected spectral information and obtain the nutrient content evaluation results extracted therefrom by the cloud service.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A near-IR (infrared) spectrophotometric system (10) for detecting the content of at least one nutrient type in a liquid food product (1), each nutrient type generating a plurality of near-IR absorptions (31) at wavelengths characteristic of said nutrient type upon exposure to near-IR radiation (21) comprising said wavelengths, the near-IR spectrophotometric system comprising:
a near-IR light source arrangement (20) comprising at least one near-IR light source (22-25) for generating the near-IR radiation; and
a near-IR detector arrangement (30) comprising at least one near-IR detector (32-35) for detecting the near-IR radiation reflected by sais liquid food product;
said near-IR spectrophotometric system being configured such that the near-IR detector arrangement exclusively detects at least one set of discrete spectral portions of said near-IR radiation, the discrete spectral portions within each set containing respective absorption wavelengths characteristic of a particular one of said at least one nutrient type.

2. The near-IR spectrophotometric system (10) of claim 1, wherein the near-IR light source arrangement (20) comprises a light source (26) arranged to generate a continuous near-IR radiation spectrum including said wavelengths and the near-IR detector arrangement (30) comprises a plurality of detectors (32-35), each of said detectors being arranged to detect one of said discrete spectral portions.

3. The near-IR spectrophotometric system (10) of claim 2, wherein said detectors (32-35) are arranged on a common substrate (36).

4. The near-IR spectrophotometric system (10) of claim 1, wherein the near-IR light source arrangement (20) comprises a plurality of light sources (22-25) each arranged to generate one of said discrete spectral portions, and the near-IR detector arrangement (30) comprises a detector (38) arranged to detect each of said discrete spectral portions.

5. The near-IR spectrophotometric system (10) of claim 4, wherein said light sources (22-25) are narrowband light emitting diodes or lasers.

6. The near-IR spectrophotometric system (10) of any of claims 1-5, wherein each discrete spectral portion has a bandwidth of less than 2 nm, preferably less than 1 nm.

7. The near-IR spectrophotometric system (10) of any of claims 1-6, wherein the total number of discrete spectral portions in each set does not exceed four.

8. The near-IR spectrophotometric system (10) of any of claims 1-7, wherein:
the at least one nutrient type comprises at least one of carbohydrates, sugar, fiber fat and protein; and
the discrete spectral portions in the set for carbohydrates respectively contain the absorption wavelengths at 860nm, 878nm, 887nm, and/or 919nm characteristic for carbohydrates;
the discrete spectral portions in the set for sugar respectively contain absorption wavelengths at 935nm, 937nm, 940nm, and/or 948nm characteristic for sugar;
the discrete spectral portions in the set for fiber respectively contain absorption wavelengths at 872nm, 891nm, 903nm, and/or 912nm characteristic for fiber;
the discrete spectral portions in the set for fat respectively contain absorption wavelengths at 829nm, 931nm, and/or 939nm; and/or characteristic for fat;
the discrete spectral portions in the set for protein respectively contain absorption wavelengths at 1028nm, and/or 1049nm characteristic for protein.

9. The near-IR spectrophotometric system (10) of any of claims 1-8, wherein the near-IR detector arrangement (30) is further adapted to detect a set of discrete further spectral portions, each of said further spectral portions containing a spectral absorption wavelength characteristic for water.

10. The near-IR spectrophotometric system (10) of any of claims 1-9, further comprising a processor (40) communicatively coupled to the near-IR detector arrangement (30) and adapted to determine the content of the at least one nutrient type from the respective intensities of the absorptions (31) measured by the near-IR detector arrangement at the wavelengths characteristic of said nutrient type.

11. The near-IR spectrophotometric system (10) of claim 10, wherein a particular measured absorption (31) comprises an overlap of a first absorption at a first wavelength characteristic of a first nutrient type and a second absorption at a second wavelength characteristic of a second nutrient type, and wherein the processor (40) is adapted to:
determine the content of the first nutrient type from measured absorptions at wavelengths characteristic of said first nutrient type other than said particular measured absorption;
scale the particular measured absorptions by subtracting the contribution of the first wavelength characteristic of a first nutrient type from said particular measured absorption using the determined content of the first nutrient type; and
determine the content of the second nutrient type from measured absorptions at wavelengths characteristic of said second nutrient type including said scaled particular measured absorption.

12. The near-IR spectrophotometric system (10) of claim 10 or 11, wherein the processor (40) is adapted to:
receive at least one of weight information and volume information for said liquid food product (1) and further determine the content of the at least one nutrient type in said liquid food product based on said received weight information and/or volume information; and/or
receive nutrient type selection information and determine the content of the at least one nutrient type in said liquid food product (1) identified in said nutrient type selection information.

13. A vessel for containing a liquid food product (1), the vessel comprising the near-IR spectrophotometric system (10) of any of claims 1-12 for detecting the nutrient content of the liquid food product within the vessel.

14. A food processing apparatus (50) for processing a liquid food product (1), the food processing apparatus comprising the vessel of claim 13, wherein said vessel is a food processing chamber (60).

15. A portable electronic device comprising the near-IR spectrophotometric system (10) of any of claims 1-12 for detecting the nutrient content of the liquid food product within a vessel.
